# FASCICULE DE BREVET EUROPEEN

(11) **EP 3 265 051 B1**
(45) Date de publication et mention de la délivrance du brevet: **24.06.2020**
(21) Numéro de dépôt: 16711877.7
(22) Date de dépôt: 07.03.2016
(51) Int. Cl.: A61K 8/37, A61K 8/40, A61K 8/49, A61Q 17/04, A61K 8/04, A61K 8/03

(54) **COMPOSITION COSMETIQUE SOLAIRE AQUEUSE DEPOURVUE DE TENSIOACTIF**
WÄSSRIGE SONNENBEZOGENE TENSIDFREIE KOSMETISCHE ZUSAMMENSETZUNG
AQUEOUS SUN-RELATED COSMETIC COMPOSITION FREE OF SURFACTANTS

(30) Priorité: 06.03.2015 FR 1551920
(43) Date de publication de la demande: 10.01.2018
(73) Titulaire: NAOS Les Laboratoires, 13855 Aix en Provence Cedex 3 (FR)
(72) Inventeur: THOREL, Jean-Noël, 75014 Paris (FR)
(74) Mandataire: Cabinet Laurent & Charras
(86) Numéro de dépôt international: PCT/FR2016/050524
(87) Numéro de publication internationale: WO 2016/142616

(56) Documents cités:
- EP-A1- 1 093 798
- EP-A1- 1 477 159
- EP-A2- 2 732 804
- DE-A1- 19 501 188
- DE-A1- 19 846 772
- DE-C1- 4 426 952
- US-B1- 6 881 415
- US-B1- 8 486 425

## Description

L'invention a pour objet une nouvelle composition cosmétique, de préférence pulvérisable, présentant une valeur SPF au moins supérieure à 10, comprenant une phase aqueuse, des filtres lipophiles et hydrophiles, la composition étant dépourvue de tensioactif et avantageusement de gélifiant.

Les radiations lumineuses de longueur d'onde comprise entre 290 nm et 400 nm, correspondant aux UVA (de longueur d'onde comprise entre 320 nm et 400 nm) et aux UVB (de longueur d'onde comprise entre 290 nm et 320 nm), sont nécessaires au bon fonctionnement de l'organisme humain. Néanmoins, elles provoquent aussi de nombreux effets négatifs. Les UVB sont ainsi plus particulièrement à l'origine d'érythèmes, de brûlures cutanées, tandis que les UVA sont a priori responsables de la formation de radicaux libres qui sont susceptibles d'induire une altération cutanée à long terme. Ces rayonnements UV doivent donc être filtrés.

Pour réfléchir ou absorber les UV, on utilise des produits dermocosmétiques contenant des filtres solaires, qui permettent d'obtenir une photoprotection vis-à-vis des UVA et des UVB. Ainsi, des compositions solaires comprenant des filtres absorbant dans les ultraviolets sont appliquées de façon topique sur les parties exposées du corps humain afin de les protéger des effets indésirables de l'exposition solaire. Traditionnellement, les produits solaires se présentent sous forme de crème, stick, lotion, ou composition pulvérisable. Les compositions pulvérisables ont l'avantage d'être beaucoup plus pratiques car l'utilisateur n'est pas obligé de les étaler manuellement une fois appliquées. Les compositions pulvérisables se prêtent donc au développement de produits « nomades» à appliquer en tout moment de la journée sur les parties exposées, ce qui améliore l'observance et donc la photoprotection.

Les filtres solaires peuvent se diviser en deux groupes, les filtres minéraux et les filtres organiques. Les filtres UV minéraux (ou physiques) également appelés "écrans" sont constitués de poudres microscopiques inertes. Ces petites particules reflètent les rayons UVA/UVB et protègent ainsi la peau du rayonnement solaire. Les deux filtres UV minéraux autorisés sont le dioxyde de titane (TiO₂) et l'oxyde de zinc (ZnO).

Les filtres UV organiques (ou chimiques) sont des substances qui absorbent les rayons UV et les transforment en radiation thermique. Les filtres solaires synthétiques assurent ainsi une protection photochimique en absorbant l'énergie des rayons ultraviolets.

Les filtres UV organiques peuvent se diviser ultérieurement en deux classes: les filtres hydrophiles et le filtres lipophiles. Les filtres hydrophiles, dont quelques exemples parmi les plus utilisés sont le Phenylbenzimidazole sulfonic acid (INCI) et le Disodium Phenyl Dibenzimidazole Tetrasulfonate (INCI), sont solubles dans les phases aqueuses, et permettent donc la formulation de produits ne contenant aucune phase grasse. Cependant, ces filtres ne permettent d'obtenir que des valeurs de SPF très basses s'ils ne sont pas associés à des filtres lipophiles. En outre, utilisés seuls, ils rendent la composition finale collante de sorte que les caractéristiques organoleptiques ne sont pas optimales. Les filtres lipophiles, dont quelques exemples sont le Bis-Ethylhexyloxyphenol Methoxyphenyl Triazine (INCI) Butyl Methoxydibenzoylmethane (INCI) et l'octocrylene (INCI), permettent d'obtenir des valeurs de SPF plus élevées. Cependant, les filtres lipophiles sont exclusivement solubles dans une matière grasse et ils ne peuvent être incorporés dans des produits exclusivement aqueux.

Dans le cas de compositions pulvérisables, les produits contenant des filtres lipophiles disponibles à ce jour sur le marché sont soit des huiles solaires anhydres, soit des émulsions. Les huiles solaires anhydres offrent une photoprotection très efficace mais sont caractérisées par un toucher très gras. Dès lors, elles ne sont donc pas adaptées à l'application sur une base déjà étalée (maquillage, fond-teint etc..). De surcroît, les huiles solaires anhydres peuvent être peu hydratantes. Les émulsions pulvérisables, de leur côté, sont hydratantes car elles contiennent une phase aqueuse, et offrent une bonne photoprotection grâce à la présence de filtres lipophiles dans la phase grasse et éventuellement, des filtres hydrophiles dans la phase aqueuse. Les émulsions pulvérisables contiennent souvent des tensioactifs, qui sont requis afin de créer une dispersion stable entre les deux phases. Elles peuvent être éventuellement stabilisées davantage encore par des gélifiants. Les tensioactifs classiques, qui sont potentiellement des irritants et photosensibilisants, peuvent entrainer de phénomènes d'intolérance cutanée ou oculaire aux produits. Les émulsions solaires sont caractérisées par un aspect translucide, blanchâtre ou opaque. De plus, les tensioactifs, de par leur propriété à disperser les corps gras, ont tendance à altérer le maquillage. Ainsi, l'application de compositions solaires classiques, qui comprennent des tensioactifs, nécessite une étape préalable de démaquillage. Les compositions solaires classiques ne peuvent donc être utilisées en application d'appoint, de façon nomade, c'est-à-dire sans préparation préalable de la peau. Or, il est bien connu que la protection conférée par les filtres solaires est limitée dans le temps. Lorsque l'exposition solaire s'effectue pendant une durée importante (plus de quelques heures), l'application de filtres protecteurs doit donc être renouvelée. Les dermatologues préconisent de renouveler l'application toutes les heures pour les produits solaires ayant des indices inférieurs à 20 SPF et toutes les deux heures pour les produits solaires ayant des indices supérieurs à 20 SPF, surtout en cas de transpiration excessive et a fortiori après un bain.

A titre d'exemple de compositions connues, la demande EP 2 092 928 décrit des hydrodispersions stables sous forme de gel. DE 10 2011 078101 décrit des compositions qui sont en fait des émulsions. EP 1 477 159 décrit des compositions pouvant se présenter sous la forme d'hydrodispersions ou d'émulsions éventuellement dépourvues d'émulsifiant. EP 1 093 798 décrit des émulsions dépourvues d'émulsifiant. DE 198 46 772 décrit des hydrodispersions, dans lesquelles la phase lipidique et la phase aqueuse sont discontinues. EP 2 732 804 décrit des hydrodispersions, définies comme des dispersions d'une phase lipidique (discontinue) dans une phase aqueuse (continue), stabilisées par un gélifiant, le sodium polyacrylate. US 6 881 415 décrit des dispersions de type eau-dans-huile, décrites comme étant des émulsions de Pickering. DE 297 14 940 décrit des solutions dépourvues d'émulsifiant, d'huile, de corps gras, de cire et de silicone.

En tout état de cause, les compositions connues se présentent de manière générale sous la forme d'émulsion ou d'hydrodispersion, lesquelles doivent nécessairement être stabilisées, que ce soit par des excipients, des particules solides ou des gélifiants. Ces compositions ne permettent donc pas de résoudre les problèmes ci-dessus évoqués.

Le problème que se propose de résoudre l'invention est celui de mettre au point une composition pulvérisable sous forme de brume, qui présente un SPF d'au moins 10 et qui ne présente pas les inconvénients ci-dessus évoqués.

Un autre problème que se propose de résoudre l'invention est celui de mettre au point une composition photoprotectrice présentant un SPF d'au moins 10, tout en contenant le moins de constituants possible.

Un troisième problème que se propose de résoudre l'invention est celui de mettre au point une composition dont la fabrication soit la plus simple possible.

Le Demandeur a constaté que de manière tout à fait surprenante, il était possible d'obtenir une composition pulvérisable présentant un SPF d'au moins 10 en combinant des filtres hydrophiles et lipophiles dans une composition, de préférence majoritairement aqueuse, en l'absence de tensioactif. En pratique, la composition se présente sous la forme d'un mélange biphasique, qu'il suffit d'agiter au moment de l'utilisation, c'est-à-dire au moment de la pulvérisation. La présence de la phase aqueuse, lorsqu'elle est majoritaire, permet de disposer d'une composition ni collante ni grasse, au surplus exempte de tensioactif et avantageusement de gélifiant, et donc de constituant additionnel sans influence sur la protection solaire et susceptible au contraire avoir un impact sur la tolérance cutané du produit. Il ne s'agit donc pas d'une émulsion, ni avantageusement d'une dispersion, stable durant son stockage.

La composition de l'invention étant exempte de tensioactif et avantageusement de gélifiant, elle ne présente pas l'inconvénient des compositions de l'art antérieur. Elle est notamment compatible avec le maquillage, c'est à dire qu'elle peut être appliquée sur la peau sur une base déjà étalée, sans nécessiter de démaquillage préalable. La composition de l'invention peut ainsi être utilisée de façon nomade, c'est dire à n'importe quel moment de la journée et sans préparation particulière de la peau de l'utilisateur.

Plus précisément, l'invention a pour objet une composition cosmétique, de préférence pulvérisable, présentant avantageusement une valeur SPF d'au moins 10, avantageusement supérieure à 15, voire 20, voire même supérieure à 25. Une telle composition comprend au moins 20%, de préférence au moins 40% en poids d'eau, au moins un filtre UV hydrophile et au moins un filtre UV lipophile, la composition étant exempte de tensioactif et avantageusement exempte de gélifiant. Avantageusement la composition cosmétique selon l'invention présente un facteur de protection dans l'ultraviolet A (FPUVA) égale ou supérieur à 9, avantageusement supérieur à 11, voire 12. Dans un mode de réalisation préféré, le produit présente un ratio FPUVA/SPF égal ou supérieur à 0,33.

Au sens de l'invention, le terme « tensioactif » désigne les composés utilisés pour stabiliser les émulsions. A titre d'exemple de tensioactifs utilisés dans le domaine cosmétique on citera notamment, à titre indicatif et nullement limitatif, les tensioactifs anioniques, comme par exemple les alkylphosphates, les alkylsarcosinates, les alkylsulphoacetates, les savons alcalins et les savons d'amines; les tensioactifs cationiques, comme par exemple les ammonium quaternaires; les tensioactifs amphotères, par exemple les alkylbetaines, les alkylamphodiacetates; les tensioactifs non ioniques comme les esters de glycérol, de sorbitan, les éthers d'alcools, les copolymères oxyde d'éthylène-oxyde de propylène à nombre d'oxyde d'éthylène faible; les émulsifiants gélifiants polymériques, par exemple les acrylates C10-30, les alkyl acrylates copolymères, ou les copolymères d'ammonium acryloyldimethyltaurate et dérivés.

Au sens de l'invention, le terme « gélifiant » désigne les composés utilisés pour obtenir des gels. A titre d'exemple de gélifiants utilisés dans le domaine cosmétique on citera notamment, à titre indicatif et nullement limitatif, les polymères vinyliques, comme les polyacrylates et le polyacrylamides, les dérivés de cellulose; les gommes/polysaccharides d'origine végétale, comme par exemple l'amidon ou le carraghénane; les polysaccharides d'origine bactérienne, fongique et/ou synthétique comme la gomme de xanthane, ou encore la gomme de Sclerotium et ses dérivés; les viscosants d'origine minérale, comme les silicates stratifiés synthétiques.

De préférence, la composition de l'invention est pulvérisable.

De préférence, la composition de l'invention a une viscosité à température ambiante comprise entre 100 et 1200 mPa.s, avantageusement comprise entre 300 et 1100 mPa.s pour une vitesse de cisaillement de 0,1 s⁻¹. Cette vitesse de cisaillement correspond à la viscosité de la composition au repos.

De préférence, la composition de l'invention a une viscosité à température ambiante comprise entre 2 et 9 mPa.s, avantageusement comprise entre 3 et 6 mPa.s pour une vitesse de cisaillement de 1000 s⁻¹, correspondant à la viscosité à l'étalement.

Les termes « température ambiante » désignent une température comprise entre 19 et 25°C, de préférence une température d'environ 20°C.

La composition de l'invention comprend deux phases non miscibles.

La composition de l'invention est une composition biphasé. Dans le contexte de l'invention, les termes « biphase» et « biphasique » désignent une composition se présentant sous la forme de deux phases non miscibles présentes chacune sous forme continue. En d'autres termes, dans la composition de l'invention, les deux phases non miscibles sont séparées et non mélangées. Ainsi, la composition de l'invention n'est pas une émulsion, ni une hydrodispersion.

Il doit être compris que, sous l'effet d'une agitation, la structure de la composition de l'invention est susceptible de changer pour former, de manière transitoire, une émulsion. Toutefois en l'absence de modifications physiques, c'est-à-dire lorsque la composition n'est pas mélangée ou agitée, la composition de l'invention est biphasique, c'est-à-dire que les phases la constituant sont complètement séparées. Ce comportement est cohérent avec celui des compositions biphasiques de manière générale. En d'autres termes, les compositions biphase se distinguent par le fait qu'elles sont constituées de deux phases qui, au repos, sont distinctes au lieu d'être émulsionnées l'une dans l'autre. En pratique, l'utilisation de compositions biphase comprend habituellement une agitation préalable à l'étalement ou la pulvérisation de la composition sur la peau afin de former une émulsion extemporanée, laquelle permet une application homogène des deux phases. En l'absence d'émulsifiants, cette émulsion extemporanée n'est pas stable, de sorte qu'au repos, les deux phases non miscibles se séparent rapidement et retrouvent leur état initial, ce phénomène étant plus connu sous le terme de "déphasage".

La formation d'une émulsion extemporanée peut le cas échéant être favorisée et/ou accélérée par l'inclusion de billes inertes (par exemple, en métal ou céramique) dans la composition ou le flacon contenant la composition. Dans un mode de réalisation avantageux, la composition de l'invention comprend des billes inertes, de préférence des billes en métal, en céramique, en plastique ou en verre. Avantageusement, il s'agit de billes en verre.

Dans le contexte de l'invention, le terme « émulsion » désigne une composition comprenant deux phases non miscibles, l'une continue dispersante, l'autre discontinue dispersée. De préférence, dans le contexte de l'invention, le terme « émulsion » fait référence à des émulsions stables dans le temps, c'est-à-dire des émulsions pour lesquelles on observe peu voire pas de séparation des deux phases non miscibles dans le temps. Le terme « émulsion » au sens de l'invention comprend donc notamment les émulsions stabilisées par des tensioactifs, et les émulsions de Pickering, lesquelles peuvent être stabilisées par des particules solides, telles que la silice colloïdale, le mica ou bien encore des particules de sulfate de baryum ou de carbonate de calcium.

Dans le contexte de l'invention, le terme « hydrodispersion » désigne une dispersion d'une phase lipidique discontinue (dite interne) dans une phase aqueuse continue (dite externe). De préférence, dans le contexte de l'invention, le terme « hydrodispersions » fait référence à des hydrodispersions stables dans le temps, c'est-à-dire des hydrodispersions pour lesquelles on observe peu voire pas de séparation des deux phases non miscibles dans le temps.

D'une façon surprenante, le Demandeur a constaté qu'une telle composition :
- est transparente avant et après application sur la peau,
- possède des caractéristiques rhéologiques proches de celles de l'eau, permettant la dispersion dans une fine brume sous forme de spray,
- permet d'obtenir un bon index de protection (facteur SPF>10),
- est pourvue de bonnes propriétés hydratantes,
- présente un bon toucher et est jugée agréable par les utilisateurs, qui la trouvent moins collante qu'une huile,
- ne laisse pas de traces sur les vêtements
- présente une viscosité très basse et très proche de celle de l'eau aux hautes vitesses de cisaillement,
- présente une bonne capacité d'étalement.

Avantageusement, la composition selon l'invention contient au moins 20%, de préférence de 50 à 80%, avantageusement entre 50 et 60% en poids d'eau.

Selon une autre caractéristique, la composition selon l'invention contient en outre au moins une huile, de préférence une huile volatile ou une huile sèche. L'huile représente en pratique entre 2 et 80%, avantageusement entre 2 et 15% en poids de la composition totale. Avantageusement, l'huile est choisie parmi les éthers, les esters, les hydrocarbures, les silicones.

Avantageusement, l'huile volatile est choisie dans le groupe comprenant isohexadecane, isododecane, isononyl isononanoate, dicaprylyl carbonate, decyl cocoate, cyclohexasiloxane, cyclopentasiloxane, dimethicone, caprylyl methicone, phenyl methicone. Avantageusement, il s'agit du cyclohexasiloxane et du cyclopentasiloxane.

Selon une caractéristique essentielle, la composition selon l'invention contient au moins un filtre UV lipophile. Le filtre lipophile est avantageusement choisi dans le groupe comprenant l'homosalate, l'octocrylene, l'ethylhexyl salicylate, l'ethylhexyl methoxycinnamate, le diethylamino hydroxybenzoyl hexyl benzoate, le butyl methoxy dibenzoylmethane, l'ethylhexyl triazone, le bis ethylhexyloxyphenol methoxyphenyl triazine, benzophenone 3, le diethylhexyl butamido triazone, le polysilicone-15 (benzylidene malonate polysiloxane), et leurs mélanges.

De préférence, le au moins un filtre lipophile représente au moins 2% en poids de la composition. De manière particulièrement préférée, le au moins un filtre lipophile représente entre 2 et 20%, avantageusement entre 14 et 20%, particulièrement avantageusement entre 15 et 20% en poids de la composition.

De préférence, la composition comprend au moins de l'ethylhexyl salicylate. Avantageusement, la composition comprend au moins de l'ethylhexyl salicylate et de l'octocrylene.

De préférence, la composition comprend au moins de l'ethylhexyl methoxycinnamate.

Selon un mode de réalisation particulièrement avantageux, la composition comprend au moins de l'ethylhexyl salicylate, de l'octocrylene, et au moins un filtre lipophile choisi dans le groupe comprenant l'homosalate, l'ethylhexyl methoxycinnamate, le diethylamino hydroxybenzoyl hexyl benzoate, le butyl methoxy dibenzoylmethane, l'ethylhexyl triazone, le bis ethylhexyloxyphenol methoxyphenyl triazine, benzophenone 3, le diethylhexyl butamido triazone, le polysilicone-15 (benzylidene malonate polysiloxane), et leur mélanges.

Selon un mode de réalisation avantageux, la composition comprend de l'homosalate, de l'octocrylene et de l'ethylhexyl salicylate. De préférence, dans ce mode de réalisation, l'homosalate et l'octocrylene représentent chacun avantageusement entre 1 et 10% en poids de la composition, tandis que l'ethylhexyl salicylate représente avantageusement entre 1 et 5% en poids de la composition.

Selon un autre mode de réalisation avantageux, la composition comprend de l'ethylhexyl methoxycinnamate, de l'octocrylene, de l'ethylhexyl salicylate et du diethylamino hydroxybenzoyl hexyl benzoate.

Dans un mode de réalisation avantageux, les filtres se présentent sous forme liquide. Dans ces conditions, ils sont mélangés à température ambiante avec la phase grasse en l'absence donc, de tout chauffage. Dans un autre mode de réalisation, ils se présentent sous forme de poudre. Dans ces conditions, ils sont mélangés avec une partie de la phase grasse préalablement chauffée.

Selon une autre caractéristique essentielle, la composition selon l'invention contient au moins un filtre UV hydrophile. Avantageusement, le au moins un filtre hydrophile est choisi dans le groupe comprenant le disodium phenyl dibenzimidazole tetrasulphonate et le phenylbenzimidazole sulphonic acid, et leurs mélanges.

De préférence, le au moins un filtre hydrophile représente entre 2 et 13% en poids de la composition, avantageusement entre 2 et 9%. De manière particulièrement préférée, le au moins un filtre hydrophile représente entre 2 et 13%, avantageusement entre 2 et 9%, particulièrement avantageusement entre 2 et 7% en poids de la composition.

Selon un mode de réalisation avantageux, la composition comprend du disodium phenyl dibenzimidazole tetrasulphonate et du phenylbenzimidazole sulphonic acid. Dans ce mode de réalisation, de préférence, le disodium phenyl dibenzimidazole tetrasulphonate représente entre 1 et 5% en poids de la composition, et le phenylbenzimidazole sulphonic acid représente entre 1 et 8% en poids de la composition.

Pour améliorer l'effet hydratant, la composition contient avantageusement un agent hydratant de préférence choisi dans le groupe comprenant le propylène glycol, le sodium hyaluronate, le sodium PCA, le propanediol, le methyl propanediol, le xylitol, la glycérine et/ou le dipropylène glycol. Avantageusement, la composition contient une combinaison de glycérine et butylène glycol. En pratique, la glycérine représente entre 1 et 10% en poids de la composition et le butylène glycol représente entre 1 et 10% en poids de la composition.

Avantageusement, la composition contient un système de conservation. Des exemples de conservateurs sont le butylène glycol, l'EDTA, le citrate trisodique, l'acide phytique, le Leuconostoc/Radish Root Ferment Filtrate (INCI), le pentylene glycol, le caprylyl glycol ou leurs associations. De préférence, l'agent conservateur est choisi dans le groupe comprenant le pentylene glycol, le caprylyl glycol, seuls ou en mélange. Chaque conservateur représente entre 0,1 et 1,0% en poids de la composition.

Avantageusement, la composition comprend en outre un acide aminé basique, avantageusement de l'arginine. Avantageusement, l'acide aminé basique représente entre 0,5 et 2%, de préférence entre 1 et 1,5% en poids de la composition.

Avantageusement, la composition contient un ou plusieurs antioxydants aptes à stabiliser les ingrédients et à prévenir le jaunissement de la solution. Avantageusement, les antioxydants sont choisis parmi le tocopheryl acetate entre 0,1 et 1,0% en poids de la composition et/ou le sodium metabisulfite entre 0,01 et 0,2% en poids de la composition.

Avantageusement, la composition comprend au moins un agent stabilisant des filtres solaires. Avantageusement, l'agent stabilisant des filtres solaires est le benzotriazolyl dodecyl p-cresol. De préférence, le benzotriazolyl dodecyl p-cresol représente entre 0,5 et 5%, de préférence environ 1% en poids de la composition.

De manière avantageuse, la composition ne contient pas d'agent colorant. De manière avantageuse, la composition ne comprend pas d'éthanol.

La composition de l'invention est particulièrement utile car très riche en filtres solaires hydrophiles et lipophiles absorbant les UVA et UVB. Elle permet donc d'éviter les dommages cosmétiques associés à l'exposition solaire, tel que le vieillissement prématuré de la peau, les rides ou les taches solaires par exemple. Un autre aspect de l'invention est donc un procédé cosmétique non thérapeutique comprenant l'application de la composition de l'invention sur la peau. De préférence, la composition de l'invention est appliquée sur la peau par pulvérisation. Avantageusement, dans le procédé de l'invention, la composition est agitée avant d'être appliquée sur la peau par pulvérisation.

La composition de l'invention permet la prévention des dommages cellulaires, tissulaires et matriciels liés au soleil, en particulier de l'érythème solaire et des dommages à l'ADN causés par les UVA et les UVB, et par suite des cancers de la peau. Un autre aspect de l'invention est donc la composition de l'invention pour son utilisation en tant que médicament, de préférence pour son utilisation pour la prévention des dommages cellulaires liés au soleil, de préférence pour la prévention de l'érythème solaire, et/ou des dommages à l'ADN causés par les UV, et/ou des cancers de la peau.

La manière dont l'invention peut être réalisée, et les avantages qui en découlent, ressortiront mieux des exemples de réalisation qui suivent, à l'appui des figures annexées.
La figure 1 correspond à une image de la composition selon l'invention fle 1 de l'exemple 1 comparée à celle d'une émulsion solaire classique. La composition selon l'invention (A) au repos est comparée à une émulsion classique (B).
La figure 2 correspond à une image la composition selon l'invention fle 1 de l'exemple 1 comparée à celle d'une émulsion solaire classique. La composition selon l'invention après agitation (A') est comparée à une émulsion classique (B).
La figure 3 représente la viscosité des compositions selon l'invention fle 1 et fle 3 de l'exemple 1 en fonction du gradient de cisaillement comparées à l'eau et à plusieurs autres produits solaires.

### 1/ Exemple de formules (fies) solaires selon l'invention

Les proportions des produits sont indiquées en pourcentage en poids par rapport au poids total de la composition.

| **Composant (INCI)** | **Fonction** | **fle 1** | **fle 2** | **fle 3** |
|---|---|---|---|---|
| Eau | | 55,15 | 40,62 | 58,33 |
| Cyclopentasiloxane | huile volatile | 7,00 | | |
| Disodium phenyl dibenzimidazole tetrasulfonate | filtre hydrophile | 6,00 | | 6,00 |
| Glycerin | | 5,00 | 15,00 | 5,00 |
| Homosalate | filtre lipophile | 5,50 | | |
| Octocrylene | filtre lipophile | 4,50 | 2,00 | 2,00 |
| Ethylhexyl salicylate | filtre lipophile | 4,00 | 4,50 | 4,50 |
| Cyclohexasiloxane | huile volatile | 4,00 | | |
| Butylene glycol | | 3,00 | | |
| Phenylbenzimidazole sulfonic acid | filtre hydrophile | 2,30 | 2,70 | |
| Arginine | | 1,30 | | 1,00 |
| Sodium hydroxide | | 0,70 | 0,43 | 0,35 |
| Pentylene glycol | conservateur | 0,50 | 0,50 | 0,50 |
| 1,2-hexanediol | | 0,30 | 0,25 | 0,25 |
| Caprylyl glycol | conservateur | 0,30 | 0,25 | 0,25 |
| Disodium EDTA | | 0,20 | 0,20 | 0,20 |
| Tocopheryl acetate | antioxydant | 0,20 | 0,10 | 0,10 |
| Sodium metabisulfite | antioxydant | 0,05 | 0,05 | 0,05 |
| Dipropylene glycol | | | 15,00 | 3,00 |
| Diethylamino hydroxybenzoyl hexyl benzoate | filtre lipophile | | 6,00 | 2,00 |
| Ethylhexyl methoxycinnamate | filtre lipophile | | 6,70 | 6,70 |
| Isononyl isononanoate | huile volatile | | 5,70 | 8,70 |
| Benzotriazolyl dodecyl p-cresol | agent stabilisant | | | 1,00 |
| Lactic acid | | | | 0,07 |

### 2/ Transparence de la composition fle 1 selon l'exemple 1

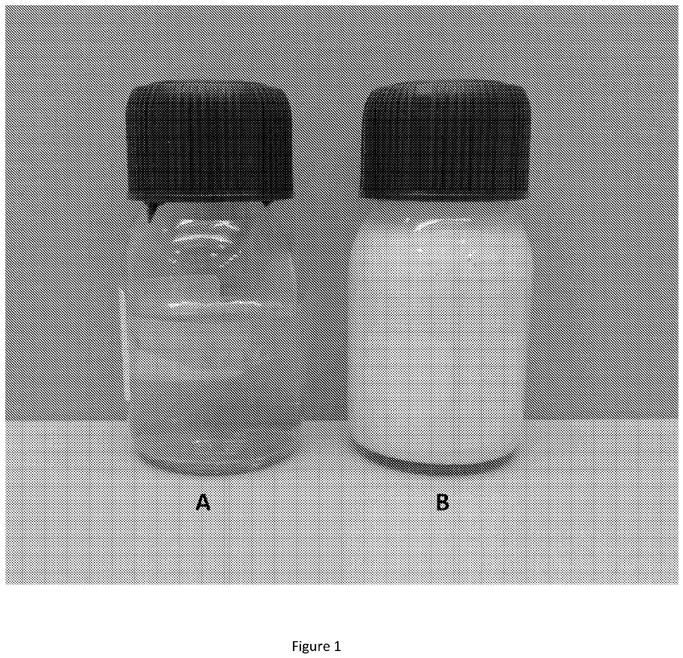

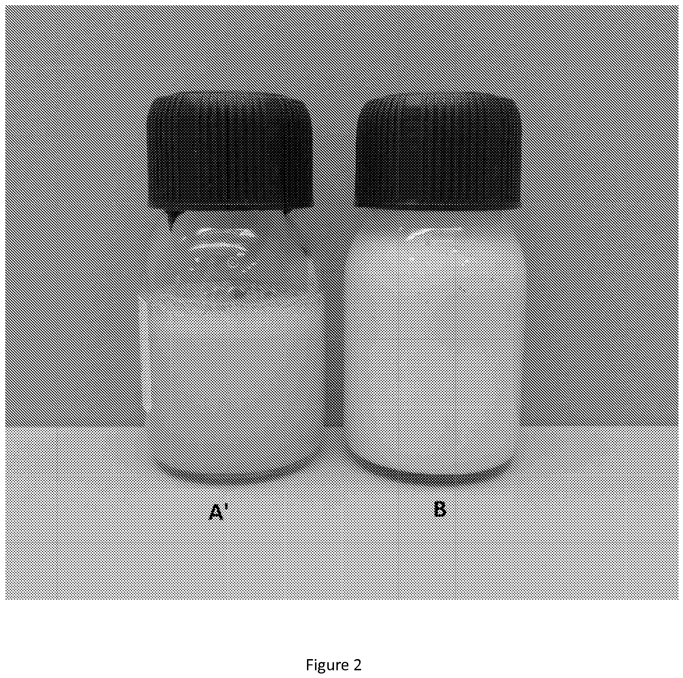

Dans ce test, la composition fle 1 selon l'exemple 1 est comparée visuellement à une émulsion solaire classique avant (figure 1) et après (figure 2) agitation.

L'émulsion solaire B est de couleur blanche. La composition selon l'invention est transparente sous la forme non mélangée A et translucide sous la forme mélangée A'.

### 3/ Etude clinique pour la détermination de l'efficacité photo-protecteur UVA (PUVA) des compositions fie 1 et fie 3 selon l'exemple 1

Une première étude a été réalisée selon la méthodologie ISO24444:2011, en utilisant la fle 1 selon l'exemple 1. Dix volontaires ont participé à l'étude.

| **1 Produit** | **Nombre de volontaires** | **FPUVA médian** | **Ecart-type** |
|---|---|---|---|
| Produit testé fle 1 de l'exemple 1 selon l'invention | 10 | 9,4 | 2,3 |
| Référence de la norme | 10 | 11 | 1,4 |

Le tableau montre que la valeur du FPUVA médian trouvé pour le produit de l'invention est de 9,4.

Une deuxième étude a été réalisée selon la méthodologie ISO24444:2006, en utilisant la fle 3 selon l'exemple 1. Quinze volontaires ont participé à l'étude.

| **Produit** | **Nombre de volontaires** | **FPUVA médian** | **Ecart-type** |
|---|---|---|---|
| Produit testé fle 3 de l'exemple 1 selon l'invention | 15 | 13,0 | 0,81 |
| Référence de la norme | 15 | 12,4 | 0,81 |

### 4/ Etude clinique pour la détermination du facteur de protection solaire (FPS ou SPF) de la composition de l'exemple 1

L'étude a été réalisée selon la méthodologie ISO24442:2010 en utilisant la fle 1 de l'exemple 1. Dix volontaires ont participé à l'étude.

| Produit | n | FPS (moyenne) |
|---|---|---|
| Produit testé (composition selon l'invention) | 10 | 30 |
| Reference de la norme | 10 | 15 |

L'étude a été réalisée selon la méthodologie ISO24442:2006 en utilisant la fle 3 de l'exemple 1. Quinze volontaires ont participé à l'étude.

| Produit | n | FPS (moyenne) |
|---|---|---|
| Produit testé (composition selon l'invention) | 15 | 35,4 |
| Reference de la norme | 15 | 15,9 |

### 5/ Exemple 5 rhéologie

### 5.1 Objectif de l'étude

Le but de l'étude est de comparer le profil rhéologique de deux formules selon l'invention (fle 1 de l'exemple 1, Lot 14B28DG127PIL00750, et fle 3 de l'exemple 1, Lot 15B35BDG18PIL00760) avec le profil rhéologique d'émulsions solaires pulvérisables (Photoderm Bronz spray SPF30 BI564V3 Lot 23741 ; La Roche Posay Anthelios dermopediatrics spray 50 Lot 10G603 ; Vichy capital soleil SPF 50 brume hydratante Lot 14L110), d'une huile solaire (Photoderm bronz huile SPF30 BI658V3 Lot 30042T) et de l'eau.

### 5.2 Matériels et méthodes

### 5.2.1 Matériel

Le laboratoire utilise un rhéomètre MCR102 de la société Anton Paar. Il est utilisée selon deux géométries: une géométrie cône-plan (CP) CP50-1/S et une plan-plan (PP) PP50/S. caractéristiques techniques :
- Roulement : air
- Couple maximal 200 mNm
- Vitesse maximale 3000 min⁻¹
- Plage de température -150 à +1000°C
- Plage de fréquence angulaire 10⁻⁷ à 628 rad/s

### 5.2.2 Tests rhéologiques

Un test d'écoulement est effectué. Le test d'écoulement permet de mesurer la viscosité de l'échantillon à différentes vitesses de cisaillement. La viscosité est relevée pour des valeurs de vitesse de cisaillement de 0,1 ; 1 ; 10 ; 100 et 1000 s⁻¹. Cette large plage de valeurs simule les différents états du produit. Les faibles vitesses de cisaillement 0,1 et 1 s⁻¹ représentent, respectivement, les conditions de stockage et de transport (produit au repos) alors que les hautes vitesses de cisaillement 100 et 1000 s⁻¹ représentent les conditions d'utilisation et d'application du produit. En particulier, la vitesse de cisaillement 1000 s⁻¹ correspond à la condition d'étalement du produit sur la peau. Le produit est laissé 1 minute au repos avant de commencer le test.

### Conditions du test :

Le test d'écoulement est effectué avec la géométrie CP à un entrefer de 0,103 mm à une température de 25°C (température ambiante).

### 5.3 Résultats

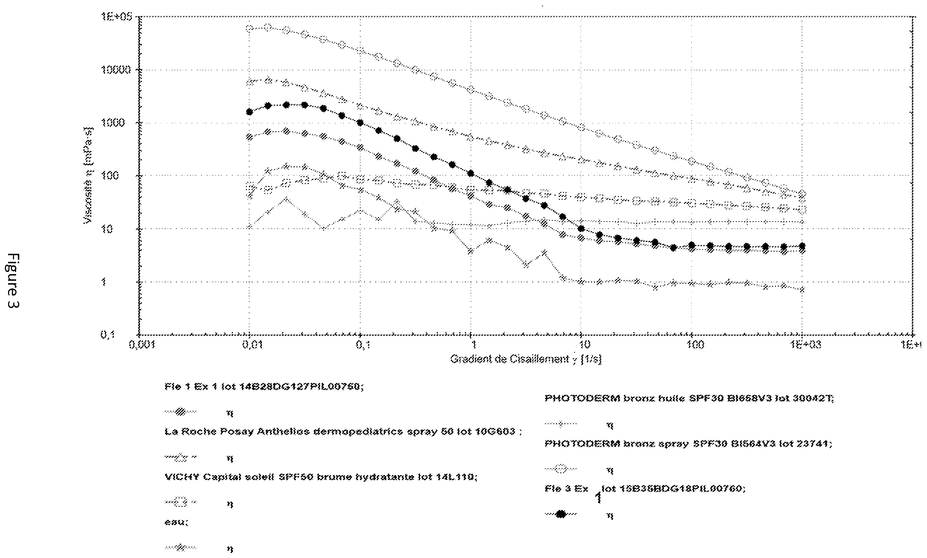

Le profil rhéologique des différentes compositions a été examiné. Afin de s'assurer de la reproductibilité du profil rhéologique, les mesures ont été effectuées 3 fois. Le produit est conditionné en pilulier en verre. Le produit est homogénéisé manuellement juste avant la mesure pour être au plus proche de l'utilisation consommateur. La figure 3 montre les résultats des profils rhéologiques.

### 5.4 Conclusions

Le produit Photoderm bronz huile SPF30 BI658V3 Lot 30042T (huile) présente un comportement rhéologique newtonien, sa viscosité est constante sur toute la gamme de cisaillement.

L'eau est un fluide newtonien de très faible viscosité.

Le produit Vichy capital soleil SPF 50 brume hydratante Lot 14L110 (émulsion E/H) se comporte comme le Photoderm bronz huile SPF30 BI658V3 Lot 30042T avec une viscosité plus élevée.

Le produit Photoderm Bronz spray SPF30 BI564V3 Lot 23741 (émulsion H/E) présente un comportement rhéologique rhéofluidifiant, sa viscosité diminue quand le gradient de cisaillement augmente.

Le produit La Roche Posay Anthelios Dermopediatrics spray 50 Lot 10G603 (émulsion H/E) se comporte comme le Photoderm Bronz spray SPF30 BI564V3 Lot 23741, mais sa viscosité est plus faible.

Les compositions selon l'invention fle 1 et fle 3 de l'exemple 1 présentent un comportement rhéologique intermédiaire entre celui des émulsions pulvérisables H/E étudiées (comportement rhéofluidifiant) et celui de l'huile solaire et de l'émulsion E/H étudiées (comportement newtonien). La viscosité à partir de 10 s⁻¹ se situe entre celle de l'huile solaire et de l'eau. Aux faibles cisaillements, elles ont un comportement rhéofluidifiant similaire à une émulsion H/E avec une viscosité plus faible. Aux fortes vitesses de cisaillement, elles tendent vers un comportement newtonien comme une huile ou l'eau. A 1000 s⁻¹ (vitesse correspondant à la condition d'étalement du produit sur la peau), les compositions selon l'invention fle 1 et fle 3 de l'exemple 1 ont une viscosité proche de celle de l'eau et plus basse que tout autre produit solaire testé.

### 6/ Test d'usage sur volontaires

### 6.1 Objectif de l'étude

Le but de l'étude était d'examiner et lister les impressions et d'évaluer l'effet hydratant du produit suite à l'application répétée de la composition fle 3 de l'exemple 1 pendant 1 semaine, dans des conditions normales d'utilisation, par des sujets adultes asiatiques.

### 6.2 Protocole de l'étude

L'étude a été réalisée en intra-individuel, chaque volontaire étant son propre témoin, à Singapour, en 2016. Les 21 volontaires recrutés ont testé la composition selon la fle 3, conditionnée dans un flacon 50 ml équipé avec une pompe panache SP22. Il a été demandé au volontaire de tester le produit sur le visage, cou et décolleté, en l'appliquant plusieurs fois dans la journée à volonté pendant une semaine. La notice suivante accompagnait le produit à tester :
« Vous utiliserez le produit de la façon suivante :
- bien agiter avant emploi jusqu'à entendre le bruit de la bille,
- fermer les yeux et brumiser d'un geste circulaire sur l'ensemble du visage et du cou en tenant le flacon à 15 cm (2 pressions),
- laisser sécher sans étaler,
- le matin, à midi et en milieu d'après-midi,
- en dernier geste de soin : seul, après une crème ou sur le maquillage,
- pendant 7 jours,
- réponse au questionnaire d'évaluation reçu par email. »

Il a été demandé aux volontaires de commencer l'application au jour 0 (J0) après la visite au centre d'investigation et de la continuer jusqu'au soir du J6. Le questionnaire d'autoévaluation devait être rempli quotidiennement, jusqu'à J7. Le questionnaire permettait d'autoévaluer l'efficacité et la tolérance cutanée du produit. Si la fréquence des applications avait été modifiée cela devait figurer dans le questionnaire. Il a également été demandé d'éviter d'appliquer le produit à tester le J7 (jour de la visite au centre d'investigation). Pour la visite du J7, l'utilisation d'un produit d'hygiène neutre a été conseillée. Il a été demandé aux volontaires de ne pas utiliser de produits similaires ou de nouveaux produits pendant toute la durée du test. Les volontaires avaient la possibilité de se maquiller durant la période du test en conservant leurs produits de maquillage habituels, ainsi que d'utiliser le produit de soin /produit d'hygiène neutre habituel.

### 6.3 Observance du protocole

Il n'a pas été observé d'incident qui puisse affecter la qualité ou l'interprétation des résultats obtenus.

### 6.4 Population étudiée

| | |
|---|---|
| Nombre de volontaires recrutés | 21 |
| Nombre de volontaires inclus | 21 |
| Nombre de volontaires sortis de l'étude | 0 |
| Nombre de volontaires dans l'analyse des résultats | 21 |

Les caractéristiques des volontaires asiatiques (81% d'origine chinoise et 19% d'origine malaisienne) sont présentées dans le tableau suivant :

| **Volontaires** | **Nature de la peau du visage** | **sensibilité** | **Volontaires sains à terrain atopique** | **Utilisateurs de maquillage (tous les jours)** | **Utilisateurs réguliers de produits FPS (SPF)** |
|---|---|---|---|---|---|
| Nombre: 21 Femmes : 21 (100 %) | Normale: 4 (19 %) Mixte | Peau du visage : 9 (43 %) | 3 (14 %) | 13 (62%) | 21 (100%) |
| Hommes : 0 (0 %) | Grasse : 6 (29 %) | | | | |
| Age moyen: 47,9 | Grasse : 3 (14 %) | | | | |
| Age min : 25 | Mixte Sèche : 5 (24 %) | | | | |
| Age max : 63 | Sèche : 2 (10 %) | | | | |
| | Très Sèche : 1 (5 %) | | | | |

### 6.5 Résultats

### 6.5.1 Appréciation des qualités cosmétiques et de l'efficacité du produit d'investigation par les volontaires

### 6.5.1.1 Questionnaire

D'après les réponses données par les volontaires aux questionnaires qui leur ont été proposés après 1 semaine d'utilisation, les conclusions suivantes peuvent être tirées: globalement, le produit a été bien apprécié pour ses qualités cosmétiques et plutôt bien apprécié pour son efficacité.

| **QUALITES COSMETIQUES (échelle en 4 points-réponse donnée par "plutôt d'accord" à "d'accord" ou par "oui*")** | **J7 (n=21)** |
|---|---|
| Après pression sur la pompe, le produit sort correctement * | 100% |
| La taille des gouttelettes est ultra-fine * | 86% |
| La texture du produit est agréable | 90% |
| La couleur du produit est agréable | 86% |
| L'odeur du produit est agréable | 86% |
| Le produit pénètre rapidement | 86% |
| Le produit est invisible à l'application | 81% |
| Le produit procure une sensation de fraîcheur à l'application | 81% |

| **EFFICACITE (échelle en 4 points-réponse donnée par "plutôt d'accord" à "d'accord")** | **J7 (n =21)** |
|---|---|
| La peau est hydratée | 90% |
| La peau est confortable | 86% |
| La peau est souple | 86% |
| La peau est douce | 90% |
| La peau est éclatante | 81% |
| La peau est protégée tout au long de la journée | 81% |
| Le produit laisse un film gras sur le visage | 52% |
| Le produit laisse un film collant sur le visage | 57% |
| Le produit laisse un film brillant sur le visage | 76% |
| Le produit laisse un film brillant sur les cheveux | 38% |

| **EFFICACITE (échelle en 4 points - réponse donnée par "plutôt d'accord" à "d'accord" ou par "oui*")** | **J7** |
|---|---|
| Le volontaire ayant appliqué de la poudre APRES l'application du produit* | 38% (8/21) |
| -> *La tenue de la poudre sur la peau est bonne* | 100% (8/8) |
| Le volontaire ayant appliqué du maquillage AVANT l'application du produit* | 52% (11/21) |
| -> *La tenue du maquillage est bonne tout au long de lajournée* | 91% (10/11) |
| -> *Le produit fixe le maquillage* | 82% (9/11) |
| > *Le maquillage tient toute la journée* | 64% (7/11) |
| *Type de maquillage* | |
| *- Fond de teint* | 55% (6/11) |
| *- Poudre* | 73% (8/11) |
| *- Correcteur* / *enlumineur* | 18% (2/11) |
| *- Fard à joues* | 27% (3/11) |

| **EFFICACITE** | **J7 (n =21)** |
|---|---|
| L'application du produit crée des peluches | 0% |

| **APPRECIATION (échelle en 2 points-réponse donnée par "oui*")** | **J7 (n=21**)** |
|---|---|
| Produit convenant au type de peau du visage du volontaire* | 76% |
| D'un point de vue cosmétique, le produit était considéré comme "bon" à "très bon | 52% |
| Le volontaire souhaiterait poursuivre l'utilisation du produit* | 76% |
| A l'issue de ce test, le sujet souhaiterait acheter le produit* | 52% |

| | |
|---|---|
| **utilisateurs réguliers de ce type de produit (produit FPS) | |

| **EN COMPARAISON AVEC LE PRODUIT HABITUEL** | **J7 (n =21)** |
|---|---|
| Par comparaison avec le produit habituellement utilisé, le volontaire trouvait sa peau "aussi bien" à "mieux" | 95% (20/21*) |

| | |
|---|---|
| *utilisateurs réguliers de ce type de produit (produit FPS) | |

| | | |
|---|---|---|
| Vol. | . Ce que le volontaire a apprécié | Ce que le volontaire n'a pas apprécié |
| 01 | Hydrate ma peau. | L'odeur n'est pas agréable |
| 02 | - | - |
| 03 | Facile à utiliser | Sensations de démangeaisons sur certaines zones : sur les joues, autour des yeux. Film collant (sensation) |
| 04 | Facile à utiliser /vaporiser (pas besoin de se servir des doigts pour appliquer le produit) | Laisse un film collant (sensation) |
| 05 | La peau est éclatante | - |
| 06 | - | Laisse un film collant sur le visage |
| 07 | Pénétration rapide. Pas de sensation de film gras | Rend les cheveux gras |
| 08 | Facile à utiliser | Trop de produit délivré lors de la vaporisation |
| 09 | Fines gouttelettes | - |
| 10 | Sans odeur | Hydrate la peau |
| 11 | - | Gras. Ne se mélange pas bien avec ma poudre libre. Ne masque pas mes imperfections. Sensation de chaleur après un moment |
| 12 | Difficile d'appliquer un SPF avec un spray | Laisse un film collant pendant un petit moment juste après application. Le vaporisateur n'est pas très bon : le produit a besoin d'être un peu étalé |
| 13 | Ma peau est hydratée | Prend du temps à sécher sur le visage |
| 14 | Ne colle pas. Hydratant | - |
| 15 | Facile à utiliser/appliquer. Pas gras. Pénétration rapide | - |
| 16 | Sans odeur. Sans couleur | Collant après application, semblable à la texture d'un écran solaire |
| 17 | Rafraîchissant. Relaxant | - |
| 18 | Rafraîchissant - | - |
| 19 | - | Gras et collant. |
| 20 | - | - |
| 21 | Vaporisation facile. Non collant, non gras | - |

### 6.5.1.2 Appréciation globale en présence du personnel de laboratoire

Selon les réponses obtenues des volontaires, les conclusions suivantes ont pu être tirées après 1 semaine d'application: le produit a été modérément bien apprécié par les volontaires pour son acceptabilité cosmétique et assez bien apprécié pour son efficacité.

| Acceptabilité cosmétique : | |
|---|---|
| - très bonne | 10% (2/21) |
| - Bonne | 43% (9/21) |
| - plutôt bonne | 10% (2/21) |
| - médiocre | 38% (8/21) |
| - bonne à très bonne | 52% (11/21) |

| Efficacité en tant que "EAU HYDRATANTE EN SPRAY AVEC FPS" : | |
|---|---|
| - très bonne | 10% (2/21) |
| - Bonne | 52% (11/21) |
| - plutôt bonne | 24% (5/21) |
| - médiocre | 14% (3/21) |
| - **bonne à très bonne** | 62% (13/21) |

### 6.5.1.3 Remontées spontanées de tolérance par les volontaires

| Signes cutanés ressentis et/ou observés: | |
|---|---|
| NON | 95% (01/21) |

| Signes oculaires/péri-oculaires ressentis et/ou observés: | |
|---|---|
| NON | 100% (21/21) |

### 6.6 Conclusions

En conclusion, le produit d'investigation, correspondant à la fle 3 selon l'exemple 1, appliqué quelques fois par jour, pendant 1 semaine, dans des conditions normales d'utilisation, sur la peau du visage, du cou et du décolleté, par 21 volontaires asiatiques adultes de sexe féminin, âgées de 25 à 63 ans, de tous types de peaux, dont 43% à la peau du visage "sensible", 62% portant tous les jours du maquillage et toutes étant utilisatrices régulières de produits FPS (et ayant l'habitude de le réappliquer au cours de la journée), a été modérément bien apprécié par les volontaires pour ses qualités cosmétiques et assez bien apprécié pour son efficacité puisque respectivement 52% et 62% d'entre elles ont jugé son acceptabilité cosmétique et son efficacité en tant que "EAU HYDRATANTE EN SPRAY CONTENANT DES FPS" comme étant "bonne" à "très bonne". En particulier, 82% des volontaires qui ont testé le produit affirment que le produit fixe le maquillage.

## Revendications

1. Composition cosmétique comprenant au moins 20% en poids d'eau, au moins une huile, au moins un filtre UV hydrophile et au moins un filtre UV lipophile,
la composition étant biphasique, sous la forme de deux phases non miscibles présentes chacune sous forme continue et exempte de tensioactif.

2. Composition selon la revendication 1, **caractérisée en ce qu'**elle est exempte de gélifiant.

3. Composition selon l'une des revendications 1 ou 2, **caractérisée en ce qu'**elle contient au moins 40%, de préférence de 50 à 80%, avantageusement entre 50 et 60% en poids d'eau.

4. Composition selon l'une des revendications 1 à 3, **caractérisée en ce que** l'huile représente entre 2 et 15% en poids de la composition totale.

5. Composition selon l'une des revendications 1 à 4, **caractérisée en ce que** l'huile est une huile volatile choisie dans le groupe comprenant isohexadecane, isododecane, isononyl isononanoate, dicaprylyl carbonate, decyl cocoate, cyclohexasiloxane, cyclopentasiloxane, dimethicone, caprylyl methicone, phenyl methicone.

6. Composition selon la revendication 5, **caractérisée en ce que** l'huile volatile est choisie dans le groupe comprenant cyclohexasiloxane et cyclopentasiloxane.

7. Composition selon l'une des revendications 1 à 6, **caractérisée en ce que** le au moins un filtre UV lipophile est choisi dans le groupe comprenant l'homosalate, l'octocrylene, l'ethylhexyl salicylate, l'ethylhexyl methoxycinnamate, le diethylamino hydroxybenzoyl hexyl benzoate, le butyl methoxy dibenzoylmethane, l'ethylhexyl triazone, le bis ethylhexyloxyphenol methoxyphenyl triazine, benzophenone 3, le diethylhexyl butamido triazone, le polysilicone-15 (benzylidene malonate polysiloxane), et leurs mélanges.

8. Composition selon la revendication 7, **caractérisée en ce que** l'homosalate et l'octocrylene représentent chacun avantageusement entre 1 et 10% en poids de la composition, tandis que l'ethylhexyl salicylate représente avantageusement entre 1% et 5% en poids de la composition.

9. Composition selon la revendication 7, **caractérisée en ce qu'**elle comprend au moins de l'ethylhexyl salicylate et de l'octocrylene.

10. Composition selon l'une des revendications 1 à 9, **caractérisée en ce que** le au moins un filtre UV hydrophile est choisi dans le groupe comprenant le disodium phenyl dibenzimidazole tetrasulphonate, le phenylbenzimidazole sulphonic acid, et leurs mélanges.

11. Composition selon la revendication 10, **caractérisée en ce que** le disodium phenyl dibenzimidazole tetrasulphonate représente entre 1 et 5% en poids de la composition, et le phenylbenzimidazole sulphonic acid représente entre 1 et 8% en poids de la composition.

12. Composition selon l'une des revendications 1 à 11, **caractérisée en ce qu'**elle contient en outre un agent hydratant choisi dans le groupe comprenant le propylène glycol, le sodium hyaluronate, le sodium PCA, le propanediol, le methyl propanediol, le xylitol, la glycérine et le butylène glycol.

13. Composition selon la revendication 12, **caractérisée en ce qu'**elle contient en outre la combinaison de glycérine et de butylène glycol.

14. Composition selon l'une des revendications 1 à 13, pour utilisation en tant que médicament.

15. Composition selon l'une des revendications 1 à 13, pour utilisation pour la prévention des dommages cellulaires liés au soleil, de préférence pour la prévention de l'érythème solaire, et/ou des dommages à l'ADN causés par les UV, et/ou des cancers de la peau.

## Patentansprüche

1. Kosmetische Zusammensetzung mit mindestens 20 Gewichts-% Wasser, mindestens einem Öl, mindestens einem hydrophilen UV- Filter und mindestens einem lipophilen UV- Filter,
die Zusammensetzung ist biphasisch, in Form zweier nicht mischbarer Phasen, die beide in kontinuierlicher Form vorhanden und frei von Tensiden sind.

2. Zusammensetzung gemäß Anspruch 1, **dadurch gekennzeichnet, dass** sie frei von Geliermitteln ist.

3. Zusammensetzung gemäß einem der Ansprüche 1 oder 2, **dadurch gekennzeichnet, dass** sie mindestens 40%, besser noch zwischen 50 und 80%, am besten zwischen 50 und 60 Gewichts-% Wasser enthält.

4. Zusammensetzung gemäß einem der Ansprüche 1 bis 3, **dadurch gekennzeichnet, dass** das Öl zwischen 2 und 15 Gewichts-% der Gesamtzusammensetzung bildet.

5. Zusammensetzung gemäß einem der Ansprüche 1 bis 4, **dadurch gekennzeichnet, dass** es sich bei dem Öl um ein ätherisches Öl handelt, ausgewählt aus der Gruppe mit Isohexadecan, Isododecan, Isononyl- Isononanoat, Dicaprylylcarbonat, Decylcocoate, Cyclohexasiloxan, Cyclopentasiloxan, Dimethicon, Caprylylmethicon, Phenylmethicon.

6. Zusammensetzung nach Anspruch 5, **dadurch gekennzeichnet, dass** das ätherische Öl ausgewählt wird aus der Gruppe mit Cyclohexasiloxan und Cyclopentasiloxan.

7. Zusammensetzung gemäß einem der Ansprüche 1 bis 6, **dadurch gekennzeichnet, dass** mindestens ein lipophiler UV- Filter ausgewählt wird aus der Gruppe mit Homosalat, Octocrylen, Ethylhexylsalicylat, Ethylhexyl-Methoxycinnamat, Diethylamino- hydroxybenzoyl- hexylbenzoat, Butyl- methoxydibenzoylmethan, Ethylhexyl- triazon, bis- ethylhexyloxyphenol- methoxyphenyltriazin, Benzophenon 3, Diethylhexylbutamidotriazon, Polysilicon-15 (Benzylidenmalonat- polysiloxan) und ihren Mischungen.

8. Zusammensetzung gemäß Anspruch 7, **dadurch gekennzeichnet, dass** das Homosalat und das Octocrylen jeweils vorteilhafterweise zwischen 1 und 10 Gewichts-% der Zusammensetzung darstellen, während das Ethylhexylsalicylat vorteilhafterweise zwischen 1% et 5 Gewichts-% der Zusammensetzung bilden.

9. Zusammensetzung gemäß Anspruch 7, **dadurch gekennzeichnet, dass** sie mindestens Ethylhexylsalicylat und Octocrylen enthält.

10. Zusammensetzung gemäß einem der Ansprüche 1 bis 9, **dadurch gekennzeichnet, dass** mindestens ein hydrophiler UV- Filter ausgewählt wird aus der Gruppe mit Dinatriumphenyl- dibenzimidazol- tetrasulphonat, Phenylbenzimidazol- Sulfonsäure und ihren Mischungen.

11. Zusammensetzung gemäß Anspruch 10, **dadurch gekennzeichnet, dass** das Dinatriumphenyl- dibenzimidazol- tetrasulphonat zwischen 1 und 5 Gewichts-% der Zusammensetzung enthält und die Phenylbenzimidazol- Sulfonsäure zwischen 1 und 8 Gewichts-% der Zusammensetzung bildet.

12. Zusammensetzung gemäß einem der Ansprüche 1 bis 11, **dadurch gekennzeichnet, dass** sie außerdem einen befeuchtenden Wirkstoff enthält, ausgewählt aus der Gruppe mit Propylenglykol, Natriumhyaluronat, Natrium PCA, Propandiol, Methyl-Propandiol, Xylitol, Glyzerin und Butylenglykol.

13. Zusammensetzung gemäß Anspruch 12, **dadurch gekennzeichnet, dass** sie außerdem eine Kombination aus Glyzerin und Butylenglykol enthält.

14. Zusammensetzung gemäß einem der Ansprüche 1 bis 13, zum Einsatz als Medikament.

15. Zusammensetzung gemäß einem der Ansprüche 1 bis 13, zum Einsatz bei der Vorbeugung von Zellschäden durch die Sonne, vorzugsweise zur Vorbeugung des Sonnenerythems und/oder von DNS- Schäden durch UV oder Hautkrebs.

## Claims

1. Cosmetic composition comprising at least 20% by weight of water, at least one oil, at least one hydrophilic UV filter and at least one lipophilic UV filter,
the composition being biphasic, in the form of two immiscible phases each present in continuous form and free from surfactant.

2. Composition according to Claim 1, **characterized in that** it is free from gelling agent.

3. Composition according to one of claims 1 to 2, **characterized in that** it contains at least 40%, preferably 50 to 80%, advantageously between 50 and 60% by weight of water.

4. Composition according to one of claims 1 to 3, **characterized in that** the oil represents between 2 and 15% by weight of the total composition.

5. Composition according to one of claims 1 to 4, **characterized in that** the oil is a volatile oil selected from the group consisting of isohexadecane, isododecane, isononyl isononanoate, dicaprylyl carbonate, decyl cocoate, cyclohexasiloxane, cyclopentasiloxane, dimethicone, caprylyl methicone, phenyl methicone.

6. Composition according to Claim 5, **characterized in that** the volatile oil is chosen from the group comprising cyclohexasiloxane and cyclopentasiloxane.

7. Composition according to one of claims 1 to 6, **characterized in that** the at least one lipophilic UV filter is chosen from the group comprising homosalate, octocrylene, ethylhexyl salicylate, ethylhexyl methoxycinnamate, diethylamino hydroxybenzoylhexylbenzoate, butyl methoxy dibenzoylmethane, ethylhexyl triazone, bis ethylhexyloxyphenol methoxyphenyl triazine, benzophenone 3, diethylhexyl butamido triazone, polysilicone-15 (benzylidene malonate polysiloxane), and mixtures thereof.

8. Composition according to claim 7, **characterized in that** the homosalate and the octocrylene each advantageously represent between 1 and 10% by weight of the composition, while the ethylhexyl salicylate advantageously represents between 1% and 5% by weight of the composition .

9. Composition according to claim 7, **characterized in that** it comprises at least ethylhexyl salicylate and octocrylene.

10. Composition according to one of claims 1 to 9, **characterized in that** the at least one hydrophilic UV filter is selected from the group consisting of disodium phenyl dibenzimidazole tetrasulphonate, phenylbenzimidazole sulphonic acid and mixtures thereof.

11. Composition according to claim 10, **characterized in that** the disodium phenyl dibenzimidazole tetrasulphonate represents between 1 and 5% by weight of the composition, and the phenylbenzimidazole sulphonic acid represents between 1 and 8% by weight of the composition.

12. Composition according to one of claims 1 to 11, **characterized in that** it additionally contains a moisturizing agent chosen from the group comprising propylene glycol, sodium hyaluronate, sodium PCA, propanediol, methyl propanediol, xylitol, glycerin and butylene glycol.

13. Composition according to claim 12, **characterized in that** it additionnally contains the combination of glycerin and butylene glycol.

14. Composition according to one of claims 1 to 13, for its use as a medicament.

15. Composition according to one of claims 1 to 13, for its use for the prevention of sun-related cell damages, preferably for preventing erythema of the sun, and/or damage to DNA caused by UV, and/or skin cancers.
